(19) Europäisches Patentamt / European Patent Office / Office européen des brevets

(11) **EP 4 146 163 B1**

(12) **EUROPÄISCHE PATENTSCHRIFT**

(45) Veröffentlichungstag und Bekanntmachung des Hinweises auf die Patenterteilung:
**03.04.2024   Patentblatt 2024/14**

(21) Anmeldenummer: **21725091.9**

(22) Anmeldetag: **07.05.2021**

(51) Internationale Patentklassifikation (IPC):
**A61K 9/00** *(2006.01)*      **A61K 31/192** *(2006.01)*
**A61K 31/122** *(2006.01)*

(52) Gemeinsame Patentklassifikation (CPC):
**A61K 9/006; A61K 31/122; A61K 31/192**

(86) Internationale Anmeldenummer:
**PCT/EP2021/062098**

(87) Internationale Veröffentlichungsnummer:
**WO 2021/224444 (11.11.2021 Gazette 2021/45)**

(54) **VERFAHREN ZUR HERSTELLUNG EINES ORALEN DÜNNFILMS UMFASSEND MIKROPARTIKEL**
PROCESS FOR PRODUCING AN ORAL THIN FILM COMPRISING MICROPARTICLES
PROCÉDÉ DE PRODUCTION D'UN FILM MINCE ORAL COMPRENANT DES MICROPARTICULES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.05.2020   DE 102020112422**

(43) Veröffentlichungstag der Anmeldung:
**15.03.2023   Patentblatt 2023/11**

(73) Patentinhaber: **LTS LOHMANN Therapie-Systeme AG**
**56626 Andernach (DE)**

(72) Erfinder:
• **LINN, Michael**
**55596 Waldböckelheim (DE)**
• **MÜLLER, Markus**
**53840 Troisdorf (DE)**
• **BAUER, Marius**
**56626 Andernach (DE)**

(74) Vertreter: **Meissner Bolte Partnerschaft mbB Patentanwälte Rechtsanwälte**
**Postfach 86 06 24**
**81633 München (DE)**

(56) Entgegenhaltungen:
**DE-A1-102017 104 277      US-A1- 2015 064 231**
**US-A1- 2017 239 172**

**Beschreibung**

Die vorliegende Erfindung betrifft ein Verfahren zur Herstellung eines oralen Dünnfilms umfassend Mikropartikel, einen oralen Dünnfilm erhältlich gemäß diesem Verfahren, sowie einen solchen oralen Dünnfilm zur Verwendung als Arzneimittel.

Ein oraler Dünnfilm umfassend Mikropartikel enthält üblicherweise Mikropartikel, die einen pharmazeutisch aktiven Wirkstoff enthalten, wobei diese Mikropartikel in einer Polymermatrix eingebettet sind. Diese Polymermatrix umfassend die Mikropartikeln löst sich nach Verabreichung des oralen Dünnfilms auf, wobei die Mikropartikel umfassend einen pharmazeutisch aktiven Wirkstoff freigesetzt werden und nach Verschlucken im Magen und/oder Darm des Patienten absorbiert werden.

DE 10 2017 104277 A1 offenbart ein transmukosales Verabreichungssystem für Idebenon.

US 2017/239172 A1 offenbart eine oral verabreichte Darreichungsform, die die Verabreichung eines Wirkstoffes lokal in der Mundhöhle für eine anhaltende Zeitspanne ermöglicht.

Bekannt sind orale Dünnfilme in Form wasserlöslicher Filme, die eine innere wasserunlösliche feste Phase in Form von Mikropartikeln umfassen, in denen sich ein Wirk- oder Geschmacksstoff befindet.

Zur Herstellung solcher Filme werden üblicherweise, wie beispielsweise in der US 2015/0064231 A1 beschrieben, Mikropartikel umfassend ein hydrophobes Polymer und einen pharmazeutisch aktiven Wirkstoff hergestellt und isoliert, um anschließend in einem zweiten Verfahrensschritt in eine hydrophile Polymermatrix eingearbeitet zu werden.

Ein solches Herstellungsverfahren hat jedoch den Nachteil, dass mindestens zwei unabhängige Verfahrensschritte zur Herstellung des oralen Dünnfilms notwendig sind. Zum anderen weisen die separat hergestellten Mikropartikel eine relativ große Partikelgröße auf, die zudem relativ schwierig einzustellen ist. Eine relativ große Partikelgröße der Mikropartikel umfassend ein hydrophobes Polymer und einen pharmazeutisch aktiven Wirkstoff hat den Nachteil, dass die Bioverfügbarkeit des Wirkstoffs herabgesetzt ist.

Die Aufgabe der vorliegenden Erfindung besteht darin, vorstehend genannte Nachteile des Standes der Technik zu beheben. Insbesondere besteht die Aufgabe der vorliegenden Erfindung darin, ein Herstellungsverfahren für einen oralen Dünnfilm umfassend Mikropartikel bereitzustellen, durch das ein oraler Dünnfilm umfassend Mikropartikel mit möglichst wenig Verfahrensschritten und möglichst einfach und kostengünstig herzustellen ist. Zudem sollen die durch dieses Verfahren hergestellten Mikropartikel in dem oralen Dünnfilm eine möglichst geringe Partikelgröße, vorzugsweise von kleiner als 100 $\mu$m, bevorzugt von kleiner als 50 $\mu$m, bevorzugt von kleiner als 20 $\mu$m, besonders bevorzugt von kleiner als 10 $\mu$m und ganz besonders bevorzugt von kleiner als 5 $\mu$m aufweisen.

Obige Aufgabe wird durch Verfahren nach Anspruch 1 gelöst, wobei das Verfahren folgende Schritte umfasst:

a) Herstellen einer Lösung umfassend ein hydrophobes Polymer, einen hydrophoben pharmazeutisch aktiven Wirkstoff und hydrophobes Lösungsmittel,

b1) Bereitstellen eines hydrophilen Polymers und Bereitstellen eines nicht mit dem Lösungsmittel aus a) mischbaren hydrophilen Lösungsmittels, oder

b2) Bereitstellen einer Lösung umfassend ein hydrophiles Polymer und ein nicht mit dem Lösungsmittel aus a) mischbares hydrophiles Lösungsmittel,

c1) Mischen der Lösung aus a) mit dem hydrophilen Polymer aus b1) gefolgt von der Zugabe des nicht mit dem Lösungsmittel aus a) mischbaren hydrophilen Lösungsmittels aus b1), um eine Emulsion zu erhalten, oder

c2) Mischen der Lösung aus a) und der Lösung aus b2), um eine Emulsion zu erhalten, und

d) Ausstreichen und Trocknen der Emulsion aus c1) oder c2), um einen oralen Dünnfilm umfassend Mikropartikel zu erhalten.

Mit dem erfindungsgemäßen einstufigen Verfahren werden die Mikropartikel nicht wie bei den bekannten Verfahren vorab separat hergestellt und in eine Polymermatrix eingearbeitet, sondern entstehen durch den Herstellungsprozess aufgrund der Tatsache, dass zwei Lösungen mit unterschiedlicher Hydrophilie/Hydrophobizität miteinander gemischt werden. Die in der Lösung a) enthaltenen Komponenten bilden kleine Tröpfchen in der hydrophiler Umgebung der Lösung b) bzw. b2) oder des Lösungsmittels aus b1). Die so hergestellte Emulsion wird anschließend ausgestrichen und getrocknet. Bei diesem Trocknungsprozess bleibt die Trennung der Phasen erhalten. Es entstehen stabile feste bzw. halbfeste Mikropartikel, die den hydrophoben pharmazeutisch aktiven Wirkstoff aber auch andere Bestandteile umfassen können.

Vorzugsweise umfasst das erfindungsgemäße Verfahren die Schritte:

a) Herstellen einer Lösung umfassend ein hydrophobes Polymer, einen hydrophoben pharmazeutisch aktiven Wirkstoff und hydrophobes Lösungsmittel,

b) Bereitstellen eines hydrophilen Polymers und Bereitstellen eines nicht mit dem Lösungsmittel aus a) mischbaren hydrophilen Lösungsmittels,

c) Mischen der Lösung aus a) mit dem hydrophilen Polymer aus b) gefolgt von der Zugabe des nicht mit dem Lösungsmittel aus a) mischbaren hydrophilen Lösungsmittels aus b), um eine Emulsion zu erhalten, und

d) Ausstreichen und Trocknen der Emulsion aus c) um einen oralen Dünnfilm umfassend Mikropartikel zu erhalten.

Vorzugsweise umfasst das erfindungsgemäße Verfahren die Schritte:

a) Herstellen einer Lösung umfassend ein hydrophobes Polymer, einen hydrophoben pharmazeutisch aktiven Wirkstoff und hydrophobes Lösungsmittel,
b) Bereitstellen einer Lösung umfassend ein hydrophiles Polymer und ein nicht mit dem Lösungsmittel aus a) mischbares hydrophiles Lösungsmittel,
c) Mischen der Lösung aus a) und der Lösung aus b), um eine Emulsion zu erhalten, und
d) Ausstreichen und Trocknen der Emulsion aus c), um einen oralen Dünnfilm umfassend Mikropartikel zu erhalten.

Vorteilhafterweise kann das hydrophobe Polymer so gewählt werden, dass die Freisetzung des mindestens einen hydrophoben pharmazeutisch aktiven Wirkstoffs gesteuert werden kann. So können beispielsweise Polymere gewählt werden, die sich in der sauren Umgebung des Magens nicht lösen, sondern erst im eher basischen Milieu des Darms auflösen.
Der Begriff "umfassen" kann auch "bestehend aus" bedeuten.
Der Begriff "Lösung" umfasst auch den Begriff "Suspension".
Das erfindungsgemäße Verfahren ist vorzugsweise dadurch gekennzeichnet, dass das Lösungsmittel in Schritt b), b1) oder b2) Wasser oder ein wässriges Lösungsmittel ist.
Wasser als Lösungsmittel ist besonders bevorzugt, da sich in diesem die Polymere lösen, die für die Bildung eines Filmes benötigt werden, der sich am Applikationsort an dem eine wässrige Umgebung vorliegt, auflösen soll. Außerdem wird ein großer Unterschied in der Polarität benötigt, um das Mischen der beiden Lösungsmittel zu verhindern, was für die Bildung der zwei Phasen benötigt wird.
Das hydrophobe Lösungsmittel umfasst vorzugsweise Ethylacetat, Methylacetat Propylacetat, flüssige Alkane, flüssige Alkene, Aromaten, Carbonsäureester, Ether und/oder Benzin.
Die Lösung aus Schritt b), b2) oder das in Schritt b1) bereitgestellte Lösungsmittel umfasst ein hydrophiles Polymer. Ein hydrophiles Polymer ist ein Polymer, das polare und/oder geladene Gruppen enthält, die das Polymer wasserlöslich machen.
Das hydrophile Polymer kann ausgewählt sein aus der Gruppe, umfassend Stärke und deren Derivate, Agar-Agar, Gelatine und andere gelbildende Proteine, Cellulose und deren Derivate, Alginsäure, Galactomannan, Carrageen und anderen pflanzliche Gummen, Pullulan und andere Di- bzw. Polysaccharide), Xanthan, Pektin und anderen Glucane, Dextran, Polyvinylpyrrolidon, Polyvinylalkohol, Poly(meth)acrylate, Polyalkylenglykole, Carboxyvinylpolymere, Polyethylenglykol-Polivinylalkohol-Copolymere (erhältlich beispielsweise unter dem Handelsnamen Kollicoat IR von BASF), Schellack, Polyvinyl-Caprolactam-Polyvinylacetat-Polyethylenglykol-Graft- und/oder Co-Polymere (erhältlich beispielsweise unter dem Handelsnamen Soloplus von BASF) und/oder Co-Polymeren davon, Chitosan, Polyoxyethylenalkylether.
In einer bevorzugten Ausführungsform umfasst das mindestens eine hydrophile Polymer Polyvinylpyrrolidon, Polyvinylalkohol, ein Cellulosederivat und/oder Co-polymere davon, Polyethylenglykol-Polyvinylalkohol-Copolymere (erhältlich beispielsweise unter dem Handelsnamen Kollicoat IR).
Diese hydrophilen Polymere haben den Vorteil, dass sie getrocknet einen dünnen stabilen Film bilden, der sich bei Applikation in einem pharmazeutisch akzeptablen Zeitraum auflöst und die Mikropartikel freigibt. Dies hat den Vorteil einer relativ schnellen Verfügbarkeit der Mikropartikel bzw. des hydrophoben pharmazeutisch aktiven Wirkstoffs sowie einer rückstandlosen Darreichung.
Die Lösung in Schritt a) umfasst mindestens ein hydrophobes Polymer.
Mit dem Begriff Hydrophobizität bezeichnet man die Eigenschaft eines Moleküls, entweder ein polares oder ein unpolares (apolares) Medium zu bevorzugen. Dabei nimmt mit zunehmendem unpolaren Charakter der Verbindung die Hydrophobizität, also die wasserabweisende Eigenschaft, eines Moleküls, zu. Hydrophobe Polymere haben entsprechend eine geringe Hydrophilie.
In einer bevorzugten Ausführungsform weist das mindestens eine hydrophobe Polymer einen logP-Wert von größer als etwa 1, bevorzugt von größer als etwa 1,5 und besonders bevorzugt von größer als etwa 2, auf.

Der n-Octanol-Wasser-Verteilungskoeffizient $K_{ow}$ (auch Schreibweisen wie Octanol/Wasser-Verteilungskoeffizient sind gebräuchlich und korrekt) ist ein dem Fachmann bekannter dimensionsloser Verteilungskoeffizient, der das Verhältnis der Konzentrationen einer Chemikalie in einem Zweiphasensystem aus n-Octanol und Wasser angibt und damit ein Maß für die Hydrophobizität bzw. Hydrophilität eines Stoffes ist. Der logP-Wert ist der dekadische Logarithmus des n-Octanol-Wasser-Verteilungskoeffizienten $K_{ow}$. Dabei gilt:

$$K_{ow} = P = \frac{c_0^{Si}}{c_w^{Si}} \qquad \text{und} \qquad \log P = \log \frac{c_0^{Si}}{c_w^{Si}} = \log c_0{}^{Si} - c_w{}^{Si}$$

mit $c_0{}^{Si}$ = Konzentration einer Chemikalie in der octanolreichen Phase und $c_w{}^{Si}$ = Konzentration einer Chemikalie in der wasserreichen Phase.

$K_{ow}$ ist größer als eins, wenn eine Substanz besser in fettähnlichen Lösungsmitteln wie n-Octanol löslich ist, und kleiner als eins wenn sie besser in Wasser löslich ist. Entsprechend ist log P positiv für lipophile und negativ für hydrophile Substanzen.

In einer besonders bevorzugten Ausführungsform umfasst das mindestens eine hydrophobe Polymer Celluloseacetatphthalat, Hypromelloseacetatsuccinat, Poly(meth)acrylat, Hypromelloseacetatphthalat, Polvinylacetatphthalat, Ethylcellulose, Celluloseacetatebutyrat, Kollophonium, Polyoxyethylenalkylethers, Polininylacetatphtalat und/oder Cellulosenitrat.

Das erfindungsgemäße Verfahren ist ferner vorzugsweise dadurch gekennzeichnet, dass das hydrophile Polymer in einer Menge zugegeben wird, so dass es 30 bis 99 Gew.-%, vorzugsweise 35 bis 90 Gew.-%, bezogen auf das Gesamtgewicht des getrockneten oralen Dünnfilms, ausmacht.

Das erfindungsgemäße Verfahren ist ferner vorzugsweise dadurch gekennzeichnet, dass das hydrophobe Polymer in einer Menge zugegeben wird, so dass es 1 bis 70 Gew.-%, vorzugsweise 10 bis 65 Gew.-%, bezogen auf das Gesamtgewicht des getrockneten oralen Dünnfilms, ausmacht.

Das erfindungsgemäße Verfahren ist ferner vorzugsweise dadurch gekennzeichnet, dass der hydrophobe pharmazeutisch aktive Wirkstoff in einer Menge zugegeben wird, so dass er 0,01 bis 40 Gew.-%, vorzugsweise 5 bis 30 Gew.-%, bezogen auf das Gesamtgewicht des getrockneten oralen Dünnfilms, ausmacht.

Der hydrophobe pharmazeutisch aktive Wirkstoff umfasst vorzugsweise einen hydrophoben Wirkstoff mit einem logP-Wert von größer als etwa 1, bevorzugt von größer als etwa 1,5 und besonders bevorzugt von größer als etwa 2, wie vorstehend definiert.

Der Begriff pharmazeutisch aktiver Wirkstoff umfasst dabei auch alle pharmazeutisch akzeptablen Salze und Solvate des jeweiligen pharmazeutisch aktiven Wirkstoffes.

Der hydrophobe Wirkstoff liegt vorzugsweise im Wesentlichen in den hydrophoben Mikropartikeln vor. Unter im Wesentlichen wird verstanden, dass der Wirkstoff zu mehr als 85 Gew.-%, bevorzugt zu mehr als 90 Gew.-% und besonders bevorzugt zu mehr als 95 Gew.-% bzw. mehr als 99 Gew.-%, bezogen auf die Gesamtmenge an Wirkstoff in dem System, in den Mikropartikeln des oralen Dünnfilms vorliegt.

Ganz besonders bevorzugt umfasst der hydrophobe pharmazeutisch aktive Wirkstoff Idebenon, Ulipristalacetat und/oder Ketoprofen.

Unter Idebenon versteht man 2-(10-hydroxydecyl)-5,6-dimethoxy-3-methyl-cyclohexa-2,5-dien-1,4-dion, das beispielsweise unter dem Handelsnamen Raxone® von Santhera Pharmaceuticals vertrieben wird.

Unter Ulipristalacetat versteht man (8S,11S,13S,14R,17R)-17-Acetoxy-11-[4-(dimethylamino)phenyl]-19-norpregna-4,9-dien-3,20-dion.

Unter Ketoprofen versteht man (RS)-2-(3-Benzoylphenyl)propionsäure.

Es können zudem vorzugsweise auch Aromastoffe und/oder ätherische Öle in den Mikropartikeln vorhanden sein.

Das erfindungsgemäße Verfahren ist außerdem vorzugsweise dadurch gekennzeichnet, dass kein Emulgator und/oder pH-Regulator zugegeben wird, was zu dem Ergebnis führt, dass in dem erhaltenen oralen Dünnfilm umfassend Mikropartikel auch kein Emulgator und/oder pH-Regulator vorhanden ist.

Emulgator ist eine Bezeichnung für Hilfsmittel zur Herstellung und zur Stabilisierung von Emulsionen, der im engeren Sinne auch als grenzflächenaktiver Stoff bzw. Tensid bezeichnet werden kann und in der Regel als öliger bis wachsartiger, aber auch pulverförmiger Stoff vorliegt.

Als pH-Regulatoren werden alle Stoffe oder Stoffgemische bezeichnet, die eine Pufferwirkung aufweisen und somit dazu dienen einen bestimmten pH-Wert einzustellen oder aufrechtzuerhalten.

Das erfindungsgemäße Verfahren ist außerdem vorzugsweise dadurch gekennzeichnet, dass es ferner die Zugabe von mindestens einem Hilfsstoff, ausgewählt aus der Gruppe, umfassend Farbstoffe, Aromastoffe, Süßstoffe, geschmacksmaskierende Mittel, Enhancer, Feuchthaltemittel, Konservierungsmittel und/oder Antioxidationsmittel, zu der Lösung a) und/oder b), oder b2) oder dem Lösungsmittel aus b1) und/oder zu der Emulsion c), c1) oder c2) umfasst.

Das erfindungsgemäße Verfahren ist ferner vorzugsweise dadurch gekennzeichnet, dass diese Hilfsstoffe in einer Menge zugegeben werden, so dass sie jeweils in einer Menge von 0,01 bis 25 Gew.-%, bezogen auf das Gesamtgewicht des getrockneten oralen Dünnfilms enthalten sind.

Das erfindungsgemäße Verfahren ist außerdem vorzugsweise dadurch gekennzeichnet, dass die Emulsion aus c), c1) oder c2) so ausgestrichen und getrocknet wird, dass die getrocknete Emulsion ein Flächengewicht von etwa 20 bis etwa 250 g/m$^2$ aufweist.

Das erfindungsgemäße Verfahren ist außerdem vorzugsweise dadurch gekennzeichnet, dass die Emulsion aus c), c1) oder c2) so ausgestrichen und getrocknet wird, dass die getrocknete Emulsion eine Schichtdicke von vorzugsweise etwa 10 μm bis etwa 500 μm, bevorzugt von etwa 50 μm bis etwa 300 μm, aufweist.

Die Emulsion wird vorzugsweise mit einem Rakel ausgestrichen.

Alternativ kann auch ein Streichkasten, ein Spiralrakelstab, eine Schlitzdüse oder ein Rollenauftragswerk zum Ausstreichen verwendet werden.

Die ausgestrichene Emulsion wird vorzugsweise in einem Trockenschrank, alternativ in einem Klimaschrank, vorzugsweise für 10 min in einem bevorzugten Temperaturbereich von 40°C bis 100 °C getrocknet.

Das erfindungsgemäße Verfahren ist außerdem vorzugsweise dadurch gekennzeichnet, dass die Emulsion aus c), c1) oder c2) gerührt bzw. anderweitig gemischt wird. Durch die Stärke des Rührens bzw. des Mischens ist es möglich die Größe der Mikropartikel zu beeinflussen. Prinzipiell gilt, dass je stärker gerührt bzw. gemischt wird, desto kleiner sind die entstehenden Mikropartikel.

In einer Ausführungsform kommt zum Rühren der Emulsion ein Turbinenrührer zum Einsatz. Geeignete Turbinenrührer sind beispielsweise bei den Firmen IKA oder Heidolph erhältlich.

Alternativ können auch Propellerrührer, Ankerrührer, Dissolver, Homogenisatoren, Kolloidmühlen, Ultraschallmischer oder Mikrofluidisiergeräte zum Rühren bzw. Mischen Einsatz gebracht werden.

Die vorgelegte Emulsion wird vorzugsweise mindestens für 15 Minuten, höchstens für 24 Stunden gerührt bzw. gemischt.

Die vorgelegte Emulsion wird vorzugsweise mit einer Umdrehungszahl von mindestens 50 U/min und höchstens 8000 U/min gerührt bzw. gemischt.

Die vorgelegte Emulsion wird vorzugsweise bei einer Temperatur von 4°C bis 50°C gerührt bzw. gemischt.

Vorzugsweise weisen die Mikropartikel eine mittlere Partikelgröße von kleiner als 100 μm, bevorzugt von kleiner als 50 μm, bevorzugt von kleiner als 20 μm, bevorzugt von kleiner als 10 μm und ganz besonders bevorzugt von kleiner als 5 μm bzw. kleiner 3 μm auf. Die mittlere Partikelgröße wird durch Lichtmikroskopie bestimmt.

Die vorliegende Erfindung betrifft ferner einen oralen Dünnfilm umfassend Mikropartikel erhältlich nach dem vorstehend beschriebenen Verfahren.

Die für das erfindungsgemäße Verfahren offenbarten Definitionen gelten auch für den oralen Dünnfilm erhältlich nach dem vorstehend beschriebenen Verfahren.

Ein durch das Verfahren erhältlicher oraler Dünnfilm umfassend Mikropartikel zeichnet sich insbesondere dadurch aus, dass die Mikropartikel eine mittlere Partikelgröße von kleiner als 100 μm, bevorzugt von kleiner als 50 μm, bevorzugt von kleiner als 20 μm, bevorzugt von kleiner als 10 μm und ganz besonders bevorzugt von kleiner als 5 μm bzw. von kleiner als 3 μm aufweisen. Die mittlere Partikelgröße wird durch Lichtmikroskopie bestimmt.

Der Vorteil derart kleiner Mikropartikel liegt in der erhöhten Bioverfügbarkeit des darin enthaltenen hydrophoben pharmazeutisch aktiven Wirkstoffs.

Schließlich betrifft die vorliegende Erfindung einen oralen Dünnfilm umfassend Mikropartikel erhältlich nach dem oben beschriebenen Verfahren zur Verwendung als Arzneimittel, insbesondere zur Behandlung von Friedreichscher-Ataxie, Duchenne-Muskeldystrophie, primärer progredienter Multiple Sklerose und/oder leberscher heriditärer Optikusneuropathie.

Die Erfindung wird nachfolgend anhand von nicht beschränkenden Beispielen erläutert.

Beispiele

Beispiel 1:

Tabelle 1

| Material | Funktion | Menge [Gew.-%] |
|---|---|---|
| Polyvinylalkohol (PVA 4-88) | Hydrophiles Polymer | 68,85 |

(fortgesetzt)

| Material | Funktion | Menge [Gew.-%] |
|---|---|---|
| Hypromelloseacetatsuccinat | Hydrophobes Polymer | 24,89 |
| Idebenon | Pharmazeutisch aktiver Wirkstoff | 6,24 |

Zur Herstellung eines erfindungsgemäßen oralen Dünnfilms der Zusammensetzung gemäß Tabelle 1 wurden 68.85 Gew.-% Polyvinylalkohol in Wasser gelöst.

Parallel wurden 24,89 Gew.-% Hypromelloseacetatsuccinat und 6,24 Gew.-% Idebenon in Methylacetat gelöst.

Anschließend wurden die Polyvinylalkohollösung und die Lösung umfassend Hypromelloseacetatsuccinat und Idebenon gemischt, um eine Emulsion zu erhalten.

Die erhaltene Emulsion wurde ausgestrichen und getrocknet.

Der erhaltene orale Dünnfilm weist eine feste hydrophile Polymermatrix auf, in der die hydrophoben Mikropartikel als separate stabile Phase vorliegen. Die Mikropartikel weisen eine mittlere Partikelgröße von kleiner als 5 $\mu$m auf (ermittelt durch Lichtmikroskopie).

Beispiel 2:

Tabelle 2

| Material | Funktion | Menge [Gew.-%] bezogen auf die trockenen Bestandteile | Menge [Gew.-%] bezogen auf die Lösung |
|---|---|---|---|
| HPMC 603 | Hydrophiles Polymer | 38,5 | 15,4 |
| HPMC 60SH60 | Hydrophiles Polymer | 0,75 | 0,30 |
| Orangen Flavor | Aroma | 3,00 | 1,20 |
| Minz Flavor | Aroma | 1,00 | 0,40 |
| Polysorbat 80 | Emulgator | 3,00 | 1,20 |
| Span 85 | Emulgator | 2,00 | 0,80 |
| Mygliol | Träger Flavor-Phase | 4,00 | 1,60 |
| Sucralose | Süßungsmittel | 1,00 | 0,40 |
| Saccarin Natrium | Süßungsmittel | 2,00 | 0,80 |
| BHT | Konservierungsmittel Flavor | 0,25 | 0,10 |
| Isomalt | Zerfallsbeschleuniger | 10,00 | 4,00 |
| Eudragit Smartseal | Hydrophobes Polymer | 9,50 | 3,80 |
| Ketoprofen | Pharmazeutisch aktiver Wirkstoff | 25,00 | 10,00 |
| Wasser | Lösemittel | -- | 45,00 |
| Methylacetat | Lösemittel | -- | 15,00 |

Zur Herstellung eines erfindungsgemäßen oralen Dünnfilms der Zusammensetzung gemäß Tabelle 2 wurden 10,00 Gew.-% Ketoprofen in 15,00 Gew.-% hydrophobem Lösemittel (Methylacetat) gelöst. Dazu wurden 3,80 Gew.-% hydrophobes Polymer (Eudragit Smartseal) gegeben und gerührt, bis das Ketoprofen gelöst war.

Zu dieser Lösung wurden die hydrophilen Polymere (Σ 15,7 %) gemäß Tabelle 2 gegeben.

Anschließend wurde 45% Wasser als hydrophiles Lösungsmittel unter schnellem Rühren zugegeben, um eine Emulsion zu erhalten.

Zu dieser Emulsion wurden die übrigen Inhaltsstoffe gegeben. Dabei wurden nacheinander HPMC 60SH50, Isomalt, Saccharin und Sucralose zugegeben. Separat wurden BHT, Miglyol, Span 85, PS80, Minze Flavor und Orangen Flavor gelöst und zu der Masse zugetropft. Es wurde mit 1,314 g Wasser nachgespült und die Masse für 5 min bei 2000 UPM zum Entgasen gerührt. Es entstand eine homogene, milchige Emulsion.Die erhaltene Emulsion wurde ausgestrichen und getrocknet.

Der erhaltene orale Dünnfilm weist eine feste hydrophile Polymermatrix auf, in der die hydrophoben Mikropartikel als separate stabile Phase vorliegen.

**Patentansprüche**

1. Verfahren zur Herstellung eines oralen Dünnfilms umfassend Mikropartikel, umfassend die Schritte:

   a) Herstellen einer Lösung umfassend ein hydrophobes Polymer, einen hydrophoben pharmazeutisch aktiven Wirkstoff und hydrophobes Lösungsmittel,
   b1) Bereitstellen eines hydrophilen Polymers und Bereitstellen eines nicht mit dem Lösungsmittel aus a) mischbaren hydrophilen Lösungsmittels, oder
   b2) Bereitstellen einer Lösung umfassend ein hydrophiles Polymer und ein nicht mit dem Lösungsmittel aus a) mischbares hydrophiles Lösungsmittel,
   c1) Mischen der Lösung aus a) mit dem hydrophilen Polymer aus b1) gefolgt von der Zugabe des nicht mit dem Lösungsmittel aus a) mischbaren hydrophilen Lösungsmittels aus b1), um eine Emulsion zu erhalten, oder
   c2) Mischen der Lösung aus a) und der Lösung aus b2), um eine Emulsion zu erhalten, und
   d) Ausstreichen und Trocknen der Emulsion aus c1) oder c2), um einen oralen Dünnfilm umfassend Mikropartikel zu erhalten.

2. Verfahren gemäß Anspruch 1, **dadurch gekennzeichnet, dass** das hydrophile Lösungsmittel Wasser oder ein wässriges Lösungsmittel ist.

3. Verfahren gemäß Anspruch 1 oder 2, **dadurch gekennzeichnet, dass** das hydrophobe Lösungsmittel Ethylacetat, Methylacetat Propylacetat, Flüssige Alkane, Flüssige Alkene, Aromaten, Carbonsäureester, Ether und/oder Benzin umfasst.

4. Verfahren gemäß irgendeinem der vorhergehenden Ansprüche, **dadurch gekennzeichnet, dass** das hydrophile Polymer ausgewählt ist aus der Gruppe, umfassend Agar-Agar, Gelatine und andere gelbildende Proteine, Cellulose und deren Derivate, Alginsäure, Galactomannan, Carrageen und anderen pflanzliche Gummen, Pullulan, Xanthan, Pektin und anderen Glucane, Dextran, Polyvinylpyrrolidon, Polyvinylalkohol, Poly(meth)acrylate, Polyalkylenglykole, Carboxyvinylpolymere, Polyethylenglykol-Polivinylalkohol-Copolymere, Schellack, Polyvinyl-Caprolactam-Polyvinylacetat-Polyethylenglykol-Graft- und/oder Co-Polymere, Chitosan, Polyoxyethylenalkylether und/oder Co-Polymeren davon.

5. Verfahren gemäß irgendeinem der vorhergehenden Ansprüche, **dadurch gekennzeichnet, dass** das hydrophobe Polymer einen logP-Wert von größer als etwa 1 aufweist.

6. Verfahren gemäß irgendeinem der vorhergehenden Ansprüche, **dadurch gekennzeichnet, dass** das hydrophobe Polymer Polymer Celluloseacetatphtalat, Hypromelloseacetatsuccinat, Poly(meth)acrylat, Hypromelloseacetatphtalat, Polvinylacetatphtalat, Ethylcellulose, Celluloseacetatebutyrat, Kollophonium, Polyoxyethylenalkylethers, Polininylacetatphtalat und/oder Cellulosenitrat umfasst.

7. Verfahren gemäß irgendeinem der vorhergehenden Ansprüche, **dadurch gekennzeichnet, dass** das hydrophile Polymer in einer Menge zugegeben wird, so dass es 30 bis 99 Gew.-%, bezogen auf das Gesamtgewicht des getrockneten oralen Dünnfilms, ausmacht.

8. Verfahren gemäß irgendeinem der vorhergehenden Ansprüche, **dadurch gekennzeichnet, dass** das hydrophobe Polymer in einer Menge zugegeben wird, so dass es 1 bis 70 Gew.-%, bezogen auf das Gesamtgewicht des getrockneten oralen Dünnfilms, ausmacht.

9. Verfahren gemäß irgendeinem der vorhergehenden Ansprüche, **dadurch gekennzeichnet, dass** der hydrophobe pharmazeutisch aktive Wirkstoff in einer Menge zugegeben wird, so dass er 0,01 bis 20 Gew.-%, bezogen auf das Gesamtgewicht des getrockneten oralen Dünnfilms, ausmacht.

**10.** Verfahren gemäß irgendeinem der vorhergehenden Ansprüche, **dadurch gekennzeichnet, dass** kein Emulgator und/oder pH-Regulator zugegeben wird.

**11.** Verfahren gemäß irgendeinem der vorhergehenden Ansprüche, **dadurch gekennzeichnet, dass** das Verfahren ferner die Zugabe von mindestens einem Hilfsstoff, ausgewählt aus der Gruppe, umfassend Farbstoffe, Aromastoffe, Süßstoffe, geschmacksmaskierende Mittel, Enhancer, Feuchthaltemittel, Konservierungsmittel und/oder Antioxidationsmittel, zu der Lösung a) und/oder zu der Lösung b) und/oder zu der Emulsion c), umfasst.

**12.** Verfahren gemäß irgendeinem der vorhergehenden Ansprüche, **dadurch gekennzeichnet, dass** der hydrophobe pharmazeutisch aktive Wirkstoff einen pharmazeutisch aktiven Wirkstoff mit einem logP von größer als 1 umfasst.

**13.** Verfahren gemäß irgendeinem der vorhergehenden Ansprüche, **dadurch gekennzeichnet, dass** die Emulsion aus c) so ausgestrichen und getrocknet wird, dass die getrocknete Emulsion ein Flächengewicht von etwa 20 bis etwa 250 g/m$^2$ aufweist.

**14.** Oraler Dünnfilm erhältlich nach dem Verfahren gemäß irgendeinem der Ansprüchen 1 bis 13.

**15.** Oraler Dünnfilm gemäß Anspruch 14 zur Verwendung als Arzneimittel.

**Claims**

**1.** A process for producing an oral thin film comprising microparticles, comprising the steps of:

a) producing a solution comprising a hydrophobic polymer, a hydrophobic pharmaceutically active ingredient and hydrophobic solvent,
b1) providing a hydrophilic polymer and providing a hydrophilic solvent immiscible with the solvent from a), or
b2) providing a solution comprising a hydrophilic polymer and a hydrophilic solvent immiscible with the solvent from a),
c1) mixing the solution from a) with the hydrophilic polymer from b1) followed by the addition of the hydrophilic solvent from b1) immiscible with the solvent from a) to obtain an emulsion, or
c2) mixing the solution from a) and the solution from b2) to obtain an emulsion, and
d) spreading and drying the emulsion from c1) or c2) to obtain an oral thin film comprising microparticles.

**2.** The process according to claim 1, **characterised in that** the hydrophilic solvent is water or an aqueous solvent.

**3.** The process according to claim 1 or claim 2, **characterised in that** the hydrophobic solvent comprises ethyl acetate, methyl acetate, propyl acetate, liquid alkanes, liquid alkenes, aromatics, carboxylic acid esters, ethers and/or gasoline.

**4.** The process according to any one of the preceding claims, **characterised in that** the hydrophilic polymer is selected from the group comprising agar-agar, gelatin and other gel-forming proteins, cellulose and derivatives thereof, alginic acid, galactomannan, carrageenan and other vegetable gums, pullulan, xanthan, pectin and other glucans, dextran, polyvinylpyrrolidone, polyvinyl alcohol, poly(meth)acrylates, polyalkylene glycols, carboxyvinyl polymers, polyethylene glycol-polyvinyl alcohol copolymers, shellac, polyvinyl caprolactam-polyvinyl acetate-polyethylene glycol graft and/or co-polymers thereof, chitosan, polyoxyethylene alkyl ether and/or co-polymers thereof.

**5.** The process according to any one of the preceding claims, **characterised in that** the hydrophobic polymer has a logP value of greater than about 1.

**6.** The process according to any one of the preceding claims, **characterised in that** the hydrophobic polymer comprises polymer cellulose acetate phthalate, hypromellose acetate succinate, poly(meth)acrylate, hypromellose acetate phthalate, polyvinyl acetate phthalate, ethyl cellulose, cellulose acetate butyrate, collophonium, polyoxyethylene alkyl ethers, polyvinyl acetate phthalate and/or cellulose nitrate.

**7.** The process according to any one of the preceding claims, **characterised in that** the hydrophilic polymer is added in an amount such that it constitutes from 30 to 99 wt.% in relation to the total weight of the dried oral thin film.

8

**8.** The process according to any one of the preceding claims, **characterised in that** the hydrophobic polymer is added in an amount such that it constitutes from 1 to 70 wt.% in relation to the total weight of the dried oral thin film.

**9.** The process according to any one of the preceding claims, **characterised in that** the hydrophobic pharmaceutically active ingredient is added in an amount such that it constitutes 0.01 to 20 wt.% in relation to the total weight of the dried oral thin film.

**10.** The process according to any one of the preceding claims, **characterised in that** no emulsifier and/or pH regulator is added.

**11.** The process according to any one of the preceding claims, **characterised in that** the process further comprises the addition of at least one excipient selected from the group comprising colourants, flavourings, sweeteners, taste-masking agents, enhancers, humectants, preservatives and/or antioxidants to the solution a) and/or to the solution b) and/or to the emulsion c).

**12.** The process according to any one of the preceding claims, **characterised in that** the hydrophobic pharmaceutically active ingredient comprises a pharmaceutically active ingredient having a logP of greater than 1.

**13.** The process according to any one of the preceding claims, **characterised in that** the emulsion from c) is spread and dried so that the dried emulsion has a basis weight of about 20 to about 250 g/m$^2$.

**14.** An oral thin film obtainable by the process according to any one of claims 1 to 13.

**15.** An oral thin film according to claim 14 for use as a medicament.

**Revendications**

**1.** Procédé de fabrication d'un film mince oral comprenant des microparticules, comprenant les étapes suivantes :

a) la fabrication d'une solution comprenant un polymère hydrophobe, une substance active pharmaceutiquement hydrophobe et un solvant hydrophobe,
b1) la fourniture d'un polymère hydrophile et la fourniture d'un solvant hydrophile non miscible avec le solvant de l'étape a), ou
b2) la fourniture d'une solution comprenant un polymère hydrophile et un solvant hydrophile non miscible avec le solvant de l'étape a),
c1) le mélange de la solution de l'étape a) avec le polymère hydrophile de l'étape b1) puis l'ajout du solvant de l'étape b1) hydrophile non miscible avec le solvant de l'étape a) afin d'obtenir une émulsion, ou
c2) le mélange de la solution de l'étape a) et de la solution de l'étape b2) afin d'obtenir une émulsion, et
d) l'étalement et le séchage de l'émulsion de l'étape c1) ou c2) afin d'obtenir un film mince oral comprenant des microparticules.

**2.** Procédé selon la revendication 1, **caractérisé en ce que** le solvant hydrophile est de l'eau ou un solvant aqueux.

**3.** Procédé selon la revendication 1 ou 2, **caractérisé en ce que** le solvant hydrophobe comprend de l'acétate d'éthyle, de l'acétate de méthyle, de l'acétate de propyle, des alcanes liquides, des alcènes liquides, des composés aromatiques, de l'ester d'acide carboxylique, de l'éther et/ou de l'essence.

**4.** Procédé selon l'une quelconque des revendications précédentes, **caractérisé en ce que** le polymère hydrophile est sélectionné à partir du groupe comprenant de l'agar-agar, de la gélatine et d'autres protéines de gélification, de la cellulose et leurs dérivés, de l'acide alginique, de la galactomannane, de la carraghénane et d'autres gommes végétales, de la pullulane, du xanthane, de la pectine et d'autres glucanes, du dextrane, de la polyvinylpyrrolidone, du polyvinylalcool, des poly(méth)acrylates, des polyalkylèneglycoles, des carboxyvinylpolymères, des copolymères de polyéthylèneglycol-polyvinylalcool, de la gomme-laque, de la greffe polyvinyle-caprolactam-polyvinylacétate-polyéthylèneglycol et/ou des copolymères, du chitosane, de l'éther alkylique de polyoxyéthylène et/ou des co-polymères de ceux-ci.

**5.** Procédé selon l'une quelconque des revendications précédentes, **caractérisé en ce que** le polymère hydrophobe présente une valeur logP supérieure à environ 1.

**6.** Procédé selon l'une quelconque des revendications précédentes, **caractérisé en ce que** le polymère hydrophobe comprend un polymère d'acétate-phtalate de cellulose, du succinate d'acétate d'hypromellose, du poly(méth)acrylate, de l'acétate-phtalate d'hypromellose, du poly-acétate phtalate de vinyle, de l'éthylcellulose, du butyrate d'acétate de cellulose, de la rosine, des éthers alkyliques de polyoxyéthylène, du polininylacétatephtalate et/ou du nitrate de cellulose.

**7.** Procédé selon l'une quelconque des revendications précédentes, **caractérisé en ce que** le polymère hydrophile est ajouté dans une quantité de sorte qu'il constitue 30 à 99 % en poids, par rapport au poids total du film mince oral séché.

**8.** Procédé selon l'une quelconque des revendications précédentes, **caractérisé en ce que** le polymère hydrophobe est ajouté dans une quantité de sorte qu'il constitue 1 à 70 % en poids, par rapport au poids total du film mince oral séché.

**9.** Procédé selon l'une quelconque des revendications précédentes, **caractérisé en ce que** la substance active pharmaceutiquement hydrophobe est ajoutée dans une quantité de sorte qu'elle constitue 0,01 à 20 % en poids, par rapport au poids total du film mince oral séché.

**10.** Procédé selon l'une quelconque des revendications précédentes, **caractérisé en ce qu'**aucun émulsifiant et/ou régulateur de pH n'est ajouté.

**11.** Procédé selon l'une quelconque des revendications précédentes, **caractérisé en ce que** le procédé comprend de plus l'ajout d'au moins un excipient sélectionné à partir du groupe comprenant des colorants, des arômes, des édulcorants, des moyens de masquage du goût, des rehausseurs, des humidifiants, des conservateurs et/ou des antioxydants, à la solution a) et/ou à la solution b) et/ou à l'émulsion c).

**12.** Procédé selon l'une quelconque des revendications précédentes, **caractérisé en ce que** la substance active pharmaceutiquement hydrophobe comprend une substance active pharmaceutique avec un logP supérieur à 1.

**13.** Procédé selon l'une quelconque des revendications précédentes, **caractérisé en ce que** l'émulsion de l'étape c) est étalée et séchée de sorte que l'émulsion séchée présente un grammage d'environ 20 à environ 250 g/m$^2$.

**14.** Film mince oral pouvant être obtenu selon le procédé selon l'une quelconque des revendications 1 à 13.

**15.** Film mince oral selon la revendication 14 pour l'utilisation comme médicament.

**IN DER BESCHREIBUNG AUFGEFÜHRTE DOKUMENTE**

**In der Beschreibung aufgeführte Patentdokumente**

- DE 102017104277 A1
- US 2017239172 A1
- US 20150064231 A1